Europäisches Patentamt

(19) European Patent Office

Office européen des brevets

(11) Publication number: 0 114 732
A2

(12) # EUROPEAN PATENT APPLICATION

(21) Application number: 84300198.3

(51) Int. Cl.³: **A 61 M 1/03**

(22) Date of filing: 13.01.84

(30) Priority: 14.01.83 US 457875

(43) Date of publication of application:
01.08.84 Bulletin 84/31.

(84) Designated Contracting States:
DE FR GB IT

(71) Applicant: AMERICAN BENTLEY, INC.
17502 Armstrong Avenue
Irvine California 92714(US)

(72) Inventor: Streator, Gordon Lucas
10350 Powderhorn Rivercourt
Fountain View California 92708(US)

(72) Inventor: Bringham, Richard
22774 Madrid Drive
El Toro California 92630(US)

(74) Representative: Nettleton, John Victor et al,
Abel & Imray Northumberland House 303-306 High
Holborn
London, WC1V 7LH(GB)

(54) Blood oxygenator heat exchanger.

(57) A blood oxygenator (200) for oxygenating venous blood thereby producing oxygenated arterial blood. The blood oxygenator includes a heat exchanger (218) for transferring heat with the blood, preferably the venous blood, prior to, or in conjunction with, oxygenation, the heat exchanger being of a coiled bellows configuration including a plurality of individual smooth rounded surface ribs (219) spaced apart by smaller diameter sections (220). Preferably, inner (110) and outer (114) scalloped members engage opposed portions of the bellows ribs providing a spiral, gently undulating blood flow path along adjacent windings of the heat exchanger coil.

EP 0 114 732 A2

./...

FIG. 1

-1-

BLOOD OXYGENATOR HEAT EXCHANGER

The invention herein relates to a blood oxygenator having a bellows configured heat exchanger with the bellows ribs engaged by scalloped members.

The history of safe and reliable blood oxygenators is relatively brief. Such oxygenators are used in openheart surgery and other operations and treatments of the body when it is necessary to establish an extracorporeal circulation system for temporarily assuming the functions of the heart and lungs of the patient. In such a system, the oxygenator operates

-2-

to perform the function usually performed by the lungs of the patient, i.e., the life-supporting transfer of oxygen into the blood and transfer carbon dioxide out of the blood. The oxygenator is used in association with a pump which performs the function of the heart to cause circulation of the blood. Thus, early versions of the oxygenator were often referred to as "heart-lung" machines. These early heart-lung machines were typically rotating discs which passed through a pool of blood, but were only partially immersed therein such that the free surface of the disc exposed the blood to oxygen and accomplished some gas transfer. After this, bag-type oxygenators were introduced which were superior to the disc oxygenators, but which left much to be desired.

A major advance occurred in the mid-1960's when the rigid (or hard shell) bubble oxygenator was developed. The history of such oxygenators had its beginnings in the device shown in Raible, et al, Patent No. 3,468,631, which is incorporated herein by reference, and they first came into clinical use with the development of the devices shown in Bentley et al, Patent Nos. 3,488,158 and 3,578,411 which have come to be known as the Bentley oxgenator. At the present time, such oxygenators are used more frequently than any other type. Among the important features of the oxygenators disclosed in the foregoing patents was the provision of a self-contained heat exchanger.

In the intervening years, some relatively minor modifications have been made in bubble oxygenators, e.g.,

-3-

those disclosed in Brumfield Patent Nos. 3,764,271 and 3,769,162. Bentley, et al, Patent Nos 3,488,158 and 3,578,411 and the aforesaid Brumfield Patents have some downward portions in the flow path of the gas blood mixture, but it is clear that they were designed to provide for initial upward flow of the gas and blood mixture in that portion of the flow path where the bubbles are formed. In addition, Fields Patent No. 3,204,631, discloses an oxygenator in which blood enters at an upper portion and oxygen enters at a lower portion such that there is an counterflow relationship with the blood initially flowing downwardly and the oxygen flowing upwardly. Further Lewin Patent No. 4,138,464 shows the desirability of alternate positioning of fluid connections for an oxygenator device.

The present invention is a further improvement of the device shown in the Bentley United States Patent No. 3,165,238, issued October 26, 1971, entitled "Oxygenator"; the Bentley, et al, United States Patent No. 3,578,411 issued May 11, 1971, entitled "Bubbler Assembly for Blood Treating Apparatus"; the Bentley, et al United States Patent No. 3,488,158 issued Janaury 6, 1970, entitled "Bubbler Assembly for Oxygenator"; and application, Serial No. 436,913, entitled "Blood Oxygenator", now abandoned, Serial No. 565,043, now Patent No. 4,058,369, entitled "An Improved Oxgenating Device", and Serial No. 54,268 entitled "Blood Oxygenator" issued as Patent No. 4,297,318 the disclosures of which are incorporated by reference herein. These

—4—

devices each represent important developments in the blood treatment art. However, since these devices temporarily assume the initial function of the heart and lungs of a patient during certain operations or other treatments of the body, further improvements are desired.

Other oxygenator configurations allow for the oxygen transfer to be accomplished across a gas permeable membrane formed either in a continuous sheet or a plurality of hollow fibers. The heat exchanger coil of this invention may be employed in conjunction with these membrane or hollow fiber oxygenators as well as hard shell bubble oxygenators.

A significant advance in the area of blood oxygenators self-contained heat exchangers occurred in the early 1960's with the development of the blood oxygenators integral heat exchanger developed by Richard DeWall, U.S. Patent No. Re. 27,100, also assigned to the assignee of this invention, Bentley Laboratories. Other significant work was performed by Dr. Frank Gollan, see pages 69-72 of Heart-Lung Bypass, Principles and Techniques of Extracorporeal Circulation (1962), Pierre Galletti, M.D. Ph.D. et al. In the following years relatvely minor modifications to the work of Dr. Gollan were made, e.g., those disclosed by Lewin Patent Nos. 4,065,264, 4,138,464 and 4,138,288, as to these three Lewin patents, see specifically Figure 15 of Patent No. 4,138,288 where an "annular ribbed" heat exchanger is shown.

-5-

The invention as claimed provides:

A blood oxygenator including:

a venous blood inlet;

an oxygenated arterial blood outlet;

an oxygen-containing gas inlet;

a heat exchanger for exchanging heat with blood;

a heat transfer fluid inlet;

said heat exchanger being further defined as including
a heat exchanger coil connected to said heat transfer fluid inlet, said heat exchanger coil being further defined as being of
bellows configuration, said bellows configuration including
a plurality of individual smooth rounded surface ribs spaced
apart by smaller diameter sections and perpendicular to the
axial centerline of the heat exchanger coil and wherein the
ratio of the outer diameter of said heat exchanger ribs to the
outer diameter of said smaller diameter sections is between
about 1.2 and about 2, thereby providing a spiral, gently
undulating blood flow path along adjacent windings of said heat
exchanger coil.

-6-

The invention as claimed also provides:

A blood oxygenator including:

a venous blood inlet;

an oxygenated. arterial blood outlet;

an oxygen-containing gas inlet;

a heat exchanger for exchanging heat with blood;

a heat transfer fluid inlet;

said heat exchanger being further defined as including a heat exchanger coil connected to said heat transfer fluid inlet of bellows configuration, said bellows configuration including a plurality of parallel individual, smooth rounded surface ribs spaced apart by smaller diameter sections, inner and outer scalloped members, said inner and outer scalloped members engaging opposed portions of said bellows ribs thereby providing a spiral gently undulating blood flow path primarily through the smaller diameter sections along adjacent windings of said heat exchanger coil wherein the ratio of the outer diameter of said heat exchanger ribs to the outer diameter of said smaller diameter sections is between about 1.2 and about 2 and the axial length of each of said heat exchanger smaller diameter sections is between about .03 and about .18 inches.

The present invention is directed to a blood oxygenator which contains means for combining oxygen-containing gas with liquid blood and a self contained heat exchanger coil. The heat exchanger coil has a bellows configuration including a number of individual smooth rounded surface ribs each spaced apart from the next by a smaller diameter coil section. Preferably inner and outer scalloped members engage opposed portions of the bellows ribs to assist in providing a spiral, gently undulating blood flow path, primarily through the coil smaller diameter sections, along adjacent windings of the heat exchanger coil.

The bellows coil is configured with certain critical tolerances such that the axial length of the smaller diameter coil sections effect a reduced blood priming volume and a low pressure resistive blood flow path between the adjacent bellows ribs while at the same time allowing for as many bellows ribs as possible per unit length. This axial length of the smaller diameter coil section should allow for proper application of a biologically compatible coating to the exterior of the coil. Additionally, the axial length of the bellows rib, preferably hollow, should be sufficient to allow adequate heat transfer fluid flow but also to allow for as many bellows ribs per unit length as possible. The internal diameter of the bellows coil must be of sufficient size as to reduce the heat transfer fluid (typically water) pressure drop through the coil. The internal diameter of the smaller diameter section should be maximized in order to reduce vibration of the heat exchanger coil.

The ratio of the outer diameter of the heat exchanger ribs to the outer diameter of the smaller diameter sections is between about 1.2 and about 2. The axial length of the heat exchanger small diameter section is between about .03 and about .18 inches. The axial length of each of the heat exchanger ribs is between about .02 and .2 inches. The ratio of the axial length of the heat exchanger smaller diameter sections to the axial length of the heat exchanger ribs is between about .1 and about 9. Further, the ratio of the heat exchanger coil internal diameter to the outer diameter of the heat exchanger ribs is between about .4 to about .8.

Ways of carrying out the invention are described in detail below with reference to drawings which illustrate three embodiments,,in which:

Figure 1 is a cross-sectional view of an oxygenator according to this invention.

Figure 2 is taken about 2-2 of Figure 1.

Figure 3 is a partial cross-sectional view of a second oxygenator according to the invention.

Figure 4 is a top view of the second oxygenator according to the invention.

Figure 5 is a cross-sectional view taken about 5-5 of Figure 4.

Figure 6 is a cross-sectional view of a third oxygenator according to this invention.

-9-

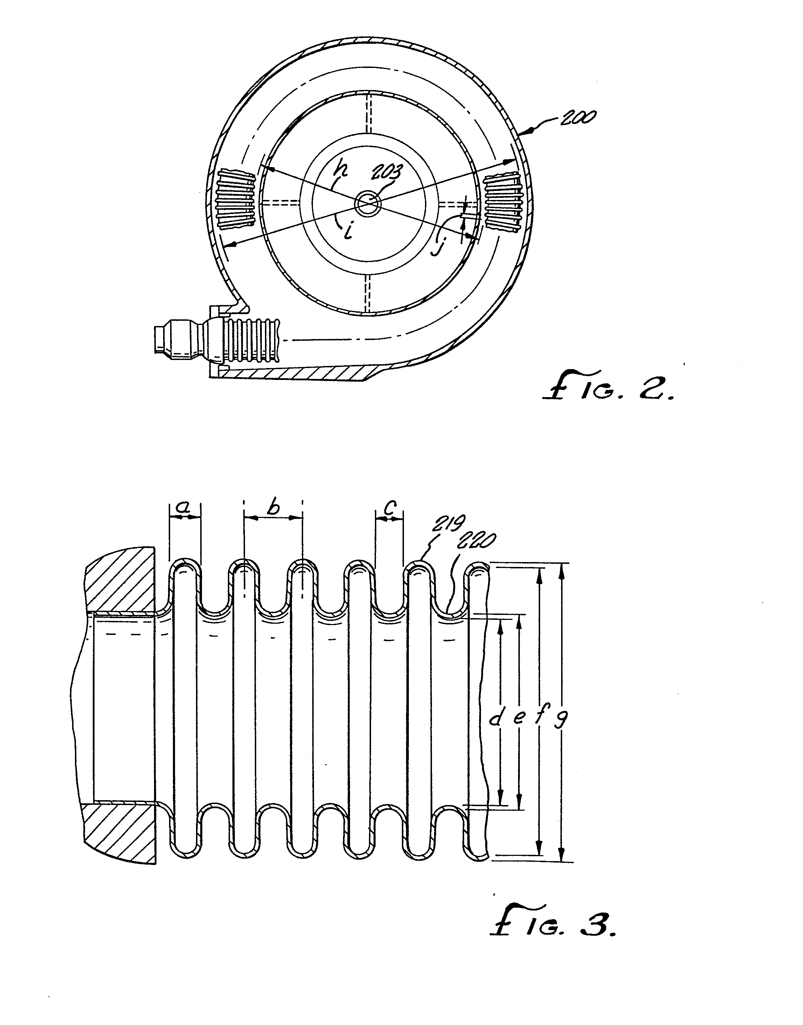

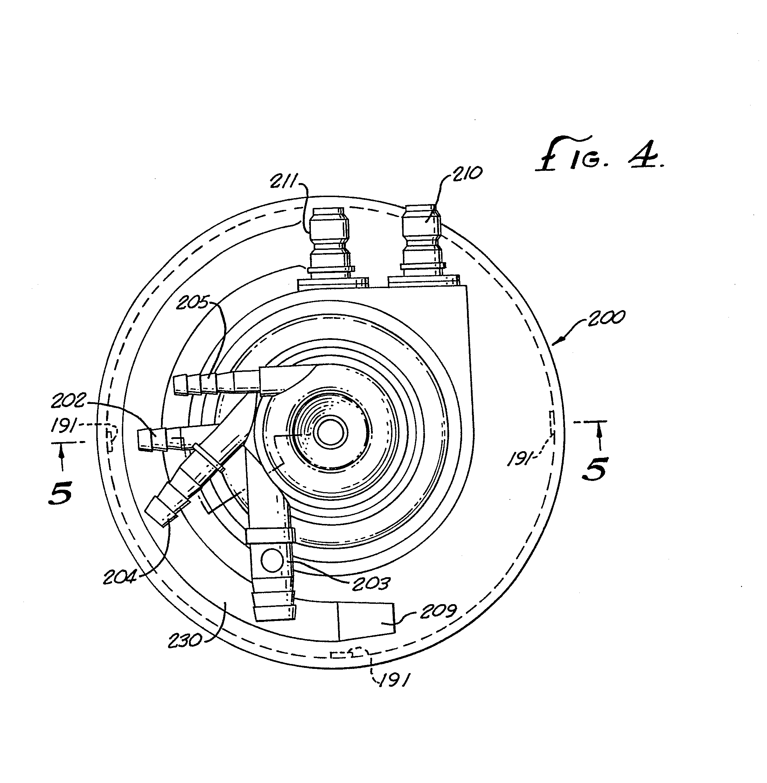

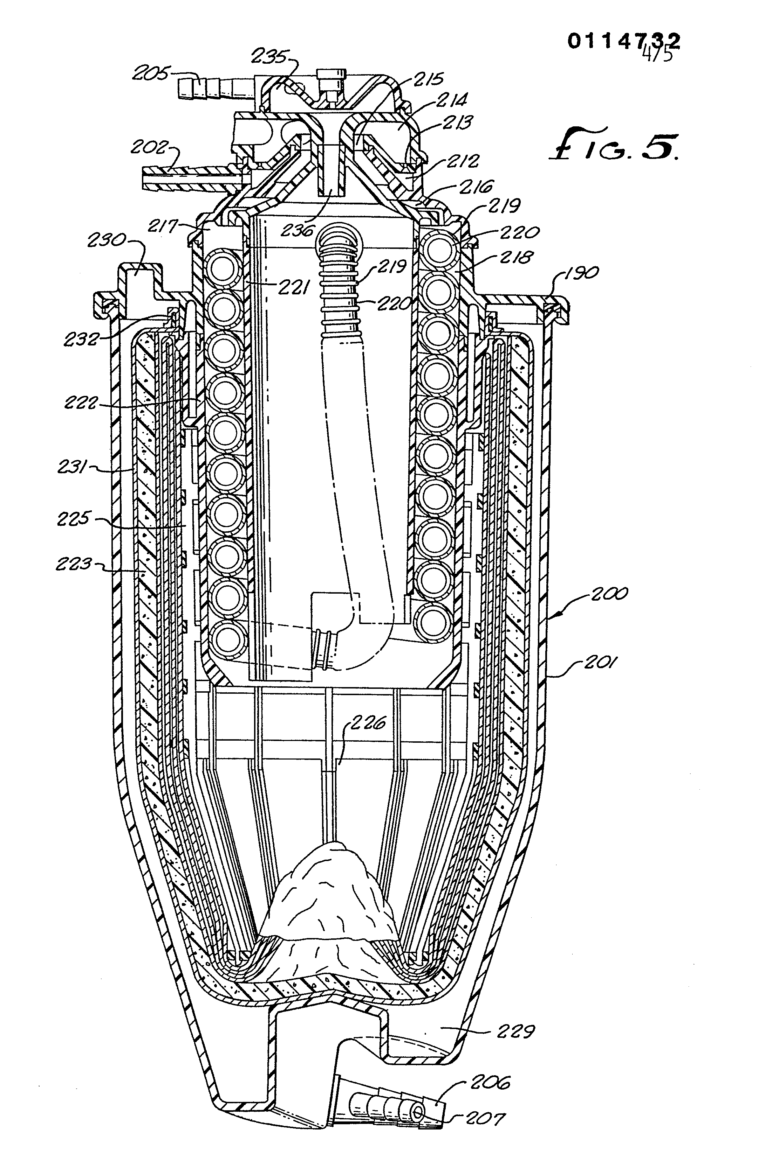

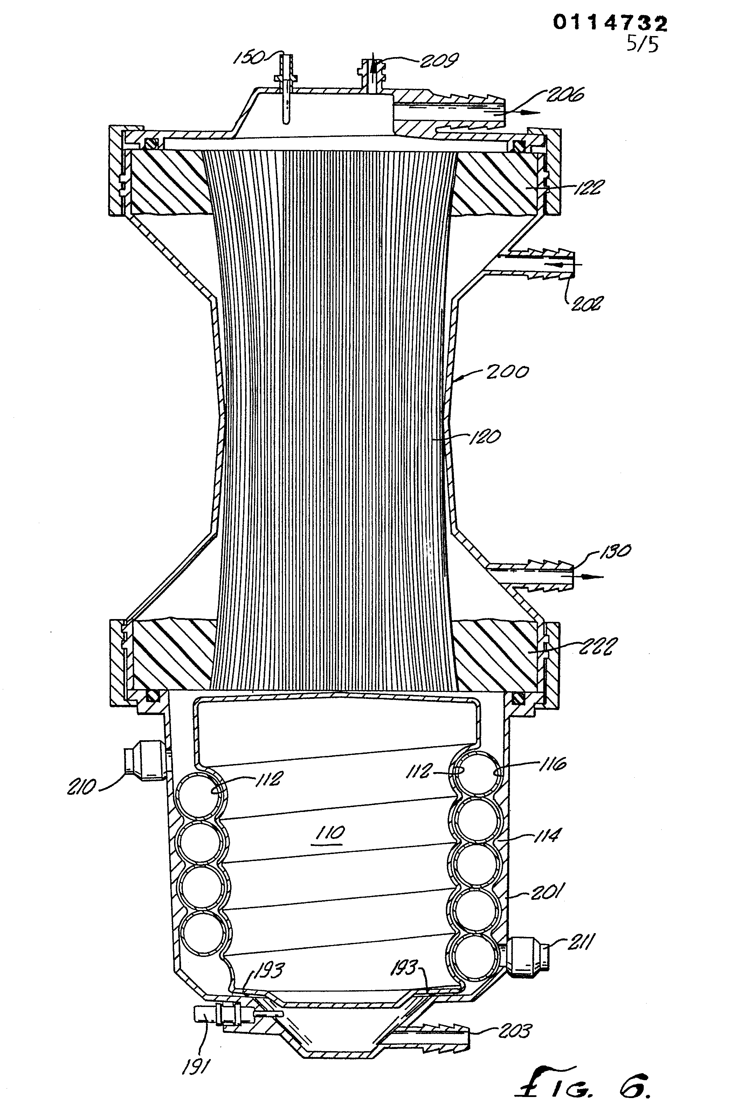

As shown in Figures 4 and 5 the oxygenator 200 of this invention includes an oxygen-containing gas inlet 202 and blood inlets 203 and 204. Inlet 205 is provided for priming as well as for optimal medical administration and, if needed, as a return inlet from the cardiotomy reservoir. A gas vent 209 is provided as are inlet 210 and outlet 211 for heat exchanger fluid. Gas inlet 202, blood inlets 203 and 204, inlet 205, vent 209 and heat exchanger fluid inlet 210 and outlet 211 are preferably located in the cap portion 190, as shown in Figure 5. This cap portion 190 of the blood oxygenator 200 is rotatably connected to an outer shell portion 201 of the oxygenator 200. Preferably, the outer shell portion 201 of the blood oxygenator includes blood outlets 206 and 207.

Referring now specifically to Figure 5, the internal construction of the oxygenator 200 is shown in greater detail. As there depicted, gas inlet 202 connects with annular chamber 212 which is bounded on its upper end by diffusion means 213. This diffusion means 213 may be of a suitable pore or perforated a pertured member, but preferably is a perforated member.

Blood inlet means 203 and 204 connect with the interior of the annular chamber 214 in a generally tangential manner. Thus, when chamber 214 is filled with blood, flowing in a spiral manner, the oxygen-containing gas is admitted to the device through inlets 202. This gas, such as oxygen or an oxygen-rich mixture, passes through inlet 202 into chamber 212 and through diffusion means 213 into the body of the blood in chamber 214.

Chamber 214 connects with annular chamber 215 and undulating distribution channel 216, the latter being conical in general shape. Channel 216 connects with annular mixing chamber 217 which is provided with heat exchange tube 218 and which contains a descending flow path for the blood. Heat exchanger-tubing 218 is a bellows tubing having a large diameter individual ribs portion 219 and a small diameter section 220. There are a plurality of descending flow paths between the walls of mixing chamber and the wall of tubing 218 formed by the convolutions.

At the lower end of chamber 217, outer wall 222 terminates approximately two-thirds of the distance from the top to the bottom of the oxygenator to permit bubbles of blood to come into contact with the defoaming means 223. Thus the elevation of the blood outlets 206 and 207 is lower than the bottom of the tortuous flow path to the mixing chamber. While several defoaming means may be used, e.g., that disclosed in Patent No. 3,468,631, it is preferred to form the defoaming material from a polyurethane foam having approximately ten to thirty pores per inch. The polyurethane foam is coated with a silicone defoaming agent. Optimally, a spacer 225 may be provided between the defoaming material 223 and wall 222. Spacer 225 may comprise a rib structure which provides open spaces therebetween.

-11-

Open spaces 226 are provided in spacer 225 which permits blood to come into contact with defoaming material reservoir 229 where liquid blood is collected.

Annular passage 230 connects with the vent means 209 so that vent gases may be exhausted from the oxygenator. A mesh sleeve 231 which may be polyester, polypropylene, polyethylene, nylon or other suitable fabric is positioned about the defoaming material 223 and is provided with elastic strap 232 to hold it in place. Port 205 connects with chamber 235 which, in turn, connects with conduit 236. Port 205 is used for priming the oxygenator and may also be used for addition of medication to the blood or for blood coming from cardiotomy reservoir.

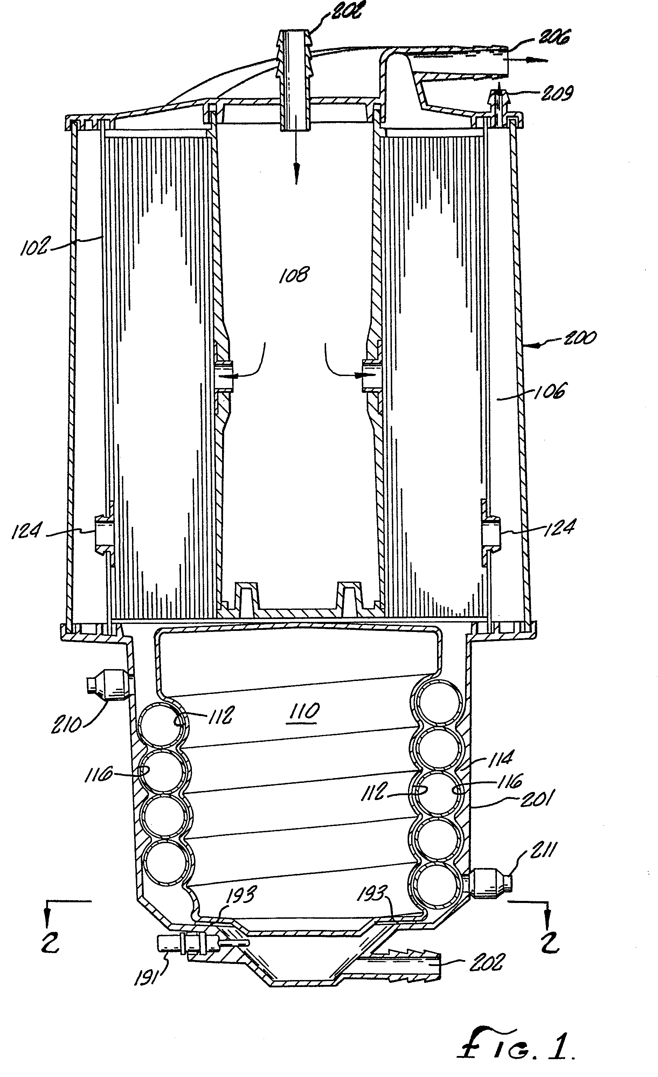

Alternate oxygenator embodiments include a membrane oxygenator shown in Figures 1 and 2 and a hollow fiber oxygenator as shown in Figure 6.

Referring now to Figure 1, the oxygenator 200 of this invention includes a blood inlet 203. A probe 191 allows for temperature measurement of the mixture of oxygen and blood. A blood outlet 206 is provided as is an inlet 210 and outlet 211 for heat transfer fluid. An oxygen-containing gas inlet 202 conveys oxygen into a hollow central chamber 108 from which it passes into the interior of a hollow membrane tube 102 which is wrapped lengthwise about the central chamber 108. The gas exits the wrapped membrane at outlets 124 into an annular exit chamber 106 and is vented through gas vent 209.

Insert 110 provides an inner scalloped member having individual scallop surfaces 112 which engage opposed portions of the heat exchange ribs 219. An outer scalloped member 114 is provided by the inner portion of outer shell 201 and has individual scallop surfaces 116 which also engage opposed portions of the heat exchanger ribs 219. Because the heat exchanger coil 218 is spirally wrapped within oxygenator 200, insert 110 may be threaded into the coil into the orientation shown in Figures 1 and 6. Spacers 193 provide a stop for the insert 110 and provide a blood passage into the area of the spirally wrapped coil 218. Blood leaves the heat exchange coil 218 and then passes along the exterior of the wrapped membrane 102 and then out through blood outlet 206.

Referring now to Figure 3, "a" refers to the axial length of each of the heat exchanger ribs 219 while "c" represents the axial length of the heat exchanger smaller diameter sections 220. Reference "b" refers to the center spacing of adjacent ribs 219. Reference "d" represents the heat exchanger coil 220 and heat exchanger smaller diametersections 220 internal diameters while "e" represents the outside diameter of the heat exchanger smaller diameter sections 220. Reference letters "f" and "g" refer to the internal and external diameters of the heat exchanger ribs 219, respectively.

Figure 2 shows the heat exchanger coil internal and external diameters "h" and "i", respectively. Also shown in Figure 2 is "j" which represents the spacing between adjacent ribs when the heat exchanger 220 is coiled as shown in Figure 2. Spacing "j" is preferably more than 20% of the axial length of the heat exchanger smaller diameter sections "b".

Referring now to Figure 6, a blood oxygenator is shown having a heat exchanger section similar to that illustrated in Figure 1. The oxygenator section differs in that oxygenation is accomplished through use of a plurality of hollow fibers 120 embedded at each end within an urethane material, or the like, 122. Blood having passed upwardly through the inside of hollow fibers 120 exits the blood oxygenator 200 through blood outlet 206. Gas vent 130 allows for the venting of gas. An oxygen-containing gas inlet 202 and gas outlet 130 are provided for circulating gas around the exterior of the hollow fibers 120. Figure 6 shows temperature probe 150 at the blood outlet region as well as probe 191 for temperature measurement at the region of the blood inlet and arterial blood sample port 209.

-14-

What is Claimed:

1. A blood oxygenator including:

a venous blood inlet;

an oxygenated arterial blood outlet;

an oxygen-containing gas inlet;

a heat exchanger for exchanging heat with blood;

a heat transfer fluid inlet;

said heat exchanger being further defined as including a heat exchanger coil connected to said heat transfer fluid inlet, said heat exchanger coil being further defined as being of bellows configuration, said bellows configuration including a plurality of individual smooth rounded surface ribs spaced apart by smaller diameter sections and perpendicular to the axial centerline of the heat exchanger coil and wherein the ratio of the outer diameter of said heat exchanger ribs to the outer diameter of said smaller diameter sections is between about 1.2 and about 2, thereby providing a spiral, gently undulating blood flow path along adjacent windings of said heat exchanger coil.

2. A blood oxygenator claimed in Claim 1 wherein the axial length of each of said heat exchanger smaller diameter sections is between about .03 and about .18 inches.

3. A blood oxygenator claimed in Claim 1 & 2 wherein the axial length of each of said heat exchanger ribs is between about .02 and about .2 inches.

-15-

4. A blood oxygenator claimed in Claim any preceding wherein the ratio of the axial length of said heat exchanger smaller diameter sections to the axial length of said heat exchanger ribs is between about .1 and about 9.

5. A blood oxygenator claimed in Claim any preceding wherein the ratio of the heat exchanger coil internal diameter to the to the external diameter of said heat exchanger coil is between about .4 and about .8.

6. A blood oxygenator including:

a venous blood inlet;

an oxygenated arterial blood outlet;

an oxygen-containing gas inlet;

a heat exchanger for exchanging heat with blood;

a heat transfer fluid inlet;

said heat exchanger being further defined as including a heat exchanger coil connected to said heat transfer fluid inlet of bellows configuration, said bellows configuration including a plurality of parallel individual, smooth rounded surface ribs spaced apart by smaller diameter sections, inner and outer scalloped members, said inner and outer scalloped members engaging opposed portions of said bellows ribs thereby providing a spiral gently undulating blood flow path primarily through the smaller diameter sections along adjacent windings of said heat exchanger coil wherein the ratio of the outer diameter of said heat exchanger ribs to the outer diameter of said smaller diameter sections is between about 1.2 and about 2 and the axial length of each of said heat exchanger smaller diameter sections is between about .03 and about .18

inches.

Fig.1.

Fig. 2.

Fig. 3.

Fig. 4.

FIG. 5.

FIG. 6.